Europäisches Patentamt

European Patent Office

Office européen des brevets

(19)

(11) Veröffentlichungsnummer: **0 017 067 B1**

(12) **EUROPÄISCHE PATENTSCHRIFT**

(45) Veröffentlichungstag der Patentschrift:
08.09.82

(51) Int. Cl.³: **C 07 D 323/06**

(21) Anmeldenummer: 80101395.4

(22) Anmeldetag: 17.03.80

(54) Verfahren zur kontinuierlichen Herstellung von Trioxan.

(30) Priorität: 30.03.79 DE 2912767

(43) Veröffentlichungstag der Anmeldung:
15.10.80 Patentblatt 80/21

(45) Bekanntmachung des Hinweises auf die Patenterteilung:
08.09.82 Patentblatt 82/36

(84) Benannte Vertragsstaaten:
AT BE CH DE FR GB IT NL SE

(56) Entgegenhaltungen:
EP-A-0 009 797
DE-B-1 135 491
DE-B-1 543 390
FR-A-1 307 851
FR-A-1 479 087
FR-A-1 495 928
FR-A-1 549 133
GB-A-1 012 372
GB-A-1 130 513
GB-A-1 413 448
US-A-2 304 080

CHEMICAL ABSTRACTS, Band 77, Nr. 14, 2. Okt. 1972,
Zusammenfassung Nr. 89072k, Seite 17 COLUMBUS
OHIO (US) & SU–A–337383 (SCIENTIFIC RESEARCH
INSTITUTE OF PLASTICS) (5. 5. 1972)

(73) Patentinhaber: HOECHST AKTIENGESELLSCHAFT,
Postfach 80 03 20, D-6230 Frankfurt/Main 80 (DE)

(72) Erfinder: Voigt, Hartmut, Schweinfurter Weg 72,
D-6000 Frankfurt am Main 70 (DE)
Erfinder: Mück, Karl-Friedrich, Dr., Wittenberger
Strasse 17, D-6200 Wiesbaden (DE)
Erfinder: Bär, Helmut, Wilhelm-Weber-Weg 4,
D-6050 Offenbach/Main (DE)
Erfinder: Mader, Herbert, Heinrich-Heine-Strasse 24,
D-6085 Nauheim (DE)
Erfinder: Burg, Karlheinz, Dr., Eichenweg 18,
D-6200 Wiesbaden (DE)
Erfinder: Sextro, Günter, Dr., Erbsenacker 43,
D-6200 Wiesbaden (DE)

(56) Entgegenhaltungen:
CHEMICAL ABSTRACTS, Band 74, Nr. 11, 15. März 1971,
Zusammenfassung Nr. 53861r, Seite 376 COLUMBUS
OHIO (US) & SU–A–278 707 (INSTITUTE OF CHEMICAL
PHYSICS, ACADEMY OF SCIENCES USSR) (21. 8. 1970)

CHEMICAL ABSTRACTS, Band 86, Nr. 11, 4. März 1977,
Zusammenfassung Nr. 72712g, Seite 625 COLUMBUS
OHIO (US) PL–A–78 137 (INSTYTUT NAWOZOW
SZTUCZNYCH) (25. 7. 1975)

CHEMICAL ABSTRACTS, Band 86, Nr. 9, 28. Februar 1977,
Zusammenfassung 55498z, Seite 448 COLUMBUS OHIO
(US) & PL–A–78 676 (INSTYTUT NAWOZOW SZTUC-
NYCH) (30. 10. 1975)

Verfahren zur kontinuierlichen Herstellung von Trioxan

Die Herstellung von Trioxan aus wässrigem Formaldehyd wird verschiedentlich in der Literatur beschrieben (vgl. Walker, Formaldehyde, Reinhold Publ. New York, 3. Auflage, 1964, Seiten 198–199). Das bei höheren Temperaturen in Gegenwart saurer Katalysatoren gebildete Trioxan wird hierbei durch Destillation aus dem Reaktionsgemisch abgetrennt. Der Synthesedampf, der ausser Trioxan, Wasser und Formaldehyd noch Nebenprodukte der Synthese enthält, wird meist entsprechend der US-A-2 304 080 in einer dem Reaktor aufgesetzten Verstärkerkolonne oder entsprechend der GB-A-1 012 372 in einer mit Verstärker und Abtriebsteil versehenen Kolonne rektifiziert. Die erhaltene trioxanreiche Fraktion wird durch Extraktion und/oder ein anderes bekanntes Trennverfahren weiter aufgearbeitet.

Es ist bekannt, dass die Zeitausbeuten (g Trioxan pro kg Formaldehyd und Stunde) bei der Trioxansynthese gering sind. Nach der DE-A-1 135 491 werden z.B. durch einfache Destillation des Reaktionsgemisches aus dem Reaktionsgefäss Zeitausbeuten von 152 g Trioxan pro kg Formaldehyd und Stunde erreicht. Die geringen Zeitausbeuten haben zur Folge, dass bei der Herstellung von Trioxan aus wässrigen Formaldehyd-Lösungen lange Verweilzeiten benötigt werden. Ausserdem sind auch grosse Reaktionsvolumina erforderlich, um technisch zufriedenstellende Raumzeitausbeuten (g Trioxan pro 1 Reaktionsvolumen und Zeit) zu erhalten.

Zur Erhöhung der Raumzeitausbeuten bei der Trioxansynthese wurde bereits vorgeschlagen,. das chemische Gleichgewicht zwischen Formaldehyd und Trioxan im Reaktionsgemisch möglichst weitgehend durch Arbeiten bei hohen Verdampfungsgeschwindigkeiten zu stören.

Diese Verfahrensweise führt jedoch zu niedrigen Trioxankonzentrationen im Synthesedampf (vgl. E. Bartholomé, Chem. Ing. Techn. *43* (1971) 597). Die Aufarbeitung solcher Reaktionsdämpfe ist wegen der niedrigen Trioxankonzentration sehr energieintensiv.

Weiterhin ist aus der DE-B-1 543 390 ein Verfahren bekannt geworden, für das als maximaler Wert der Zeitausbeute 1 090 g Trioxan pro kg Formaldehyd und Stunde genannt werden. Nach diesem Verfahren wird wässrige Formaldehyd-Lösung wie üblich in Gegenwart von sauren Katalysatoren in einem Umlaufverdampfer zum Sieden erhitzt, und die trioxanreichen Synthesedämpfe werden dann über eine, dem Reaktor aufgesetzte Kolonne entfernt. In dieser Kolonne wird dem Synthesedampf Reaktionsflüssigkeit entgegengeleitet, die sich im chemischen Gleichgewicht befindet. Hierdurch wird erreicht, dass die Trioxankonzentration der Synthesedämpfe auch bei hohen Verdampfungsgeschwindigkeiten den Gleichgewichtswert erreicht, der sonst nur bei niedrigen Verdampfungsgeschwindigkeiten erhalten wird und dem Verteilungsgleichgewicht von Trioxan in gasförmiger und flüssiger Phase entspricht.

Nachteil des Verfahrens gemäss der DE-B-1 543 390 ist jedoch, dass Zusatzaggregate benötigt werden, die eine erhebliche Vergrösserung des eigentlichen Reaktorvolumens und damit eine Erniedrigung der Raumzeitausbeuten bedeuten. So besteht in dem beschriebenen Verfahren der mit der Reaktionsflüssigkeit in Kontakt kommende Verfahrensteil aus dem eigentlichen Reaktor, einem Verdampfer, einer Pumpe, einer langen Rohrleitung, gegebenenfalls mit Verweilgefäss und einer Kolonne. All diese Teile müssen aus Materialien gefertigt sein, die gegenüber einer etwa 100° C heissen sauren, z.B. schwefelsauren Formaldehyd-Lösung beständig sind. Die generellen Nachteile von korrosionsbeständigen Reaktoren sind in der DE-B-2 103 687 beschrieben. Berechnet man nun die Raumzeitausbeuten, dann ergeben sich wesentlich geringere Werte als für die in der DE-B-1 543 390 angegebenen Zeitausbeuten.

In der DE-A-2 428 719 wird ein Verfahren zur Abtrennung von Trioxan aus wässrigen Lösungen, die Trioxan zusammen mit Formaldehyd enthalten, beschrieben, wonach 5 bis 15 Gew.-% der Lösungen bei Temperaturen unter 100° C unter vermindertem Druck und bei Verweilzeiten von weniger als 1 Minute abdestilliert werden und anschliessend das Trioxan aus dem Destillat isoliert wird. Nachteilig an dieser Verfahrensweise sind die geringen Zeitausbeuten.

Schliesslich ist vorgeschlagen worden, hohe Raumzeitausbeuten an Trioxan auch bei hohen Durchsätzen dadurch zu erreichen, dass in einem Zwangsumlaufreaktor der Quotient aus verdampfter Reaktionsmischung und aus der durch den Verdampfer umlaufenden Reaktionsmischung 0,001 bis 0,04 beträgt. Um diese Werte zu verwirklichen, benötigt man zwischen Reaktor und Verdampfer Pumpen mit entsprechend grosen Förderleistungen.

Die vorliegende Erfindung betrifft nun ein Verfahren zur kontinuierlichen Herstellung von Trioxan aus wässrigen Formaldehyd-Lösungen in Gegenwart saurer Katalysatoren in einem Umlaufreaktor mit Verdampfer bei Verweilzeiten zwischen 2 und 240 Minuten, das dadurch gekennzeichnet ist, dass das den Verdampfer verlassende Dampf-Flüssigkeitsgemisch unter den Flüssigkeitsspiegel der Reaktionsmischung im Reaktor eingeleitet wird und der dampfförmige Produktstrom aus dem Reaktor austritt.

Weiterhin hat die Erfindung ein kontinuierliches Verfahren zur gleichzeitigen Herstellung von Trioxan und cyclischen Formalen zum Gegenstand, wobei eine wässrige Formaldehyd-Lösung, die mindestens ein Diol und/oder mindestens ein Epoxyd enthält, in der vorstehenden Weise behandelt wird.

Nach dem erfindungsgemässen Verfahren werden in überraschender Weise Trioxankonzentrationen im Synthesedampf auch bei hohen Verdampfungsgeschwindigkeiten erhalten, die dem Gleichgewichtswert entsprechen.

Die Trimerisierungsreaktion von Formaldehyd zu Trioxan erfolgt in an sich bekannter Weise durch Umsatz von wässrigen, im allgemeinen 30–80%igen, vorzugsweise 40–70%igen Formaldehyd-Lösungen, gegebenenfalls unter Zusatz der bekannten Antischaummittel, in Gegenwart der hierfür bekannten sauren Katalysatoren, wie Mineralsäuren, starken organischen Säuren oder einer, in seiner katalytischen Aktivität entsprechenden Menge eines anderen sauren Katalysators. Als saure Katalysatoren, die weniger flüchtig als das Reaktionsgemisch sein müssen, haben sich beispielsweise Schwefelsäure, Phosphorsäure, p-Toluolsulfonsäure oder saure Ionenaustauscher als brauchbar erwiesen. Die Menge ist nicht kritisch und beträgt in der Regel 2 bis 25%, vorzugsweise 2 bis 10%.

Falls nach der erfindungsgemässen Variante ein Gemisch aus Trioxan und mindestens einem cyclischen Formal hergestellt werden soll, werden der wässrigen Formaldehyd-Lösung zweckmässigerweise 1 bis 25 Gew.-%, vorzugsweise 2 bis 15 Gew.-%, bezogen auf Formaldehyd, an mindestens einem Diol und/oder mindestens einem Epoxyd zugesetzt.

Die hierfür in Frage kommenden Diole sind vor allem 1,2 Diole, 1,3 Diole und $\alpha,\omega$-Diole. Ebenso können statt der 1,2-Diole auch die entsprechenden Epoxyde oder Gemische aus beiden eingesetzt werden. Vorzugsweise werden Diole verwendet, deren cyclische Formale Siedepunkte von kleiner als 150° C haben und/oder mit Wasser niedrigsiedende Azeotrope (< 150° C) bilden oder wasserdampfflüchtig sind. Als geeignet haben sich z.B. Äthylenglykol, Äthylenoxyd, Propylenglykol-1,2, Propylenoxyd, Propylenglykol-1,3, Butandiol-1,2, Butandiol-1,3, Butandiol-1,4 und Buten (3)-diol-1,2 erwiesen. Vorzugsweise werden erfindungsgemäss dabei Äthylenglykol bzw. Äthylenoxyd, Propylenglykol-1,2 und Butandiol-1,4 eingesetzt und besonders bevorzugt Äthylenglykol bzw. Äthylenoxyd.

Die Reaktion wird erfindungsgemäss in einem Umlaufreaktor, vorzugsweise mit Zwangsförderung, der mit einem Verdampfer ausgestattet ist, durchgeführt. Besonders geeignet sind Zwangsumlaufverdampfer, bestehend aus Reaktionskessel, Pumpe und Verdampfer, wie sie zum Beispiel in Ullmann, Bd. 1 (1951), 3. Auflage, Seite 533–537 beschrieben sind. Die Förderleistung der Pumpe ist dabei nicht kritisch; sie richtet sich u.a. nach dem gewünschten Verdampfergrad.

Die Verweilzeit der Reaktionsmischung im Reaktionssystem beträgt 2 bis 240 Minuten, vorzugsweise 5 bis 120 Minuten und besonders bevorzugt 15 bis 60 Minuten. Die Temperaturen der Reaktionsmischung liegen je nach Druck zwischen 50° C und 150° C, vorzugsweise 95° C und 110° C.

Das Reaktionsprodukt bestehend aus Trioxan, Formaldehyd und Wasser sowie ggf. cyclischen Formalen wird im Verdampfer verdampft. Hierzu kann bei Normaldruck, unter vermindertem Druck,

beispielsweise zwischen 150 und 950 mbar oder unter Überdruck, beispielsweise 1–4 bar, gearbeitet werden. Vorzugsweise wird bei Normaldruck gearbeitet.

Der Verdampfergrad (Quotient aus verdampfter Produktmenge und durch den Verdampfer umlaufende Produktmenge x 100) der Reaktionsmischung im Verdampfer liegt beim erfindungsgemässen Verfahren, beispielsweise zwischen 0,1 bis 25%, vorzugsweise zwischen 1 und 20% und besonders vorteilhaft zwischen 6 und 14%. Entsprechend dem erfindungsgemässen Verfahren wird das den Verdampfer verlassende Flüssigkeitsdampf-Gemisch mittels eines Tauchrohres in den Reaktionskessel unterhalb des Flüssigkeitsspiegels der Reaktionsmischung eingeleitet. Dieses Abtauchen in die Reaktionsmischung sollte so tief wie möglich vorgenommen werden, damit ein optimaler Stoffaustausch und eine optimale Rührung des Reaktorinhalts (Mammutpumpenprinzip) gewährleistet ist. Die Eintauchtiefe sollte beispielsweise 20–80%, vorzugsweise 30–70% des verwendeten Reaktorfüllstandes betragen. Dabei müssen Abtauchtiefe und Art sowie Anordnung der Pumpe (soweit vorhanden) so aufeinander abgestimmt sein, dass keine Kavitation auftreten kann.

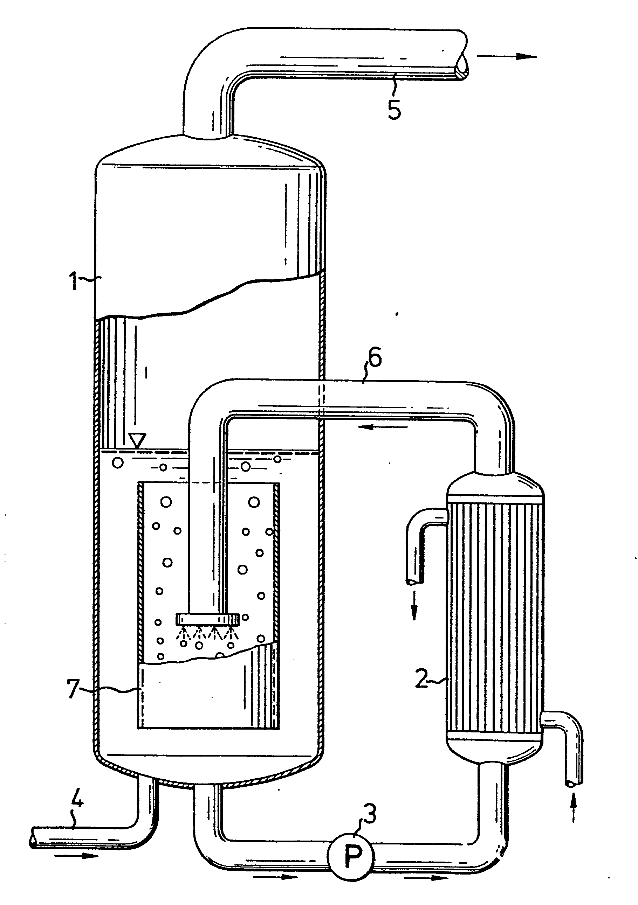

Zur Verbesserung des Stoffaustausches zwischen flüssiger und gasförmiger Phase und des Mammutpumpenprinzips kann eine zusätzliche Durchmischung und Begasung des Reaktorinhalts durch Installation der hierfür bekannten Trennelemente (Verteiler) in den Reaktor, wie zentrisch um das Tauchrohr angeordneten Metallbleche, vorzugsweise in Zylinderform entsprechend Abb. 1, erfolgen.

Der das Reaktorsystem verlassende Synthesedampf wird in üblicher Weise entweder als Dampf oder als Kondensat mittels einer Rektifikation, wie z.B. in der GB-A-1 012 372 beschrieben, angereichert. Die anfallende trioxanreiche Fraktion, die ggf. auch cyclische Formale enthält, kann dann z.B. durch Extraktion mit einem mit Wasser nichtmischbaren Lösungsmittel für Trioxan und ggf. cyclische Formale, wie Methylenchlorid und anschliessnder Neutralisation und fraktionierte Destillation oder Kristallisation gereinigt werden. Auch andere bekannte Trennverfahren können hierfür Einsatz finden, wie beispielsweise in Process Economics Program, Standford Institut Report 23 (1967), S. 181 oder in der DE-A-1 570 335 beschrieben.

Das erfindungsgemässe Verfahren ist auch verfahrenstechnisch besonders vorteilhaft, da hierbei ausser z.B. einer zwischen Reaktor und Verdampfer installierten Pumpe keine Vergrösserung des Reaktionsraums durch Zusatzaggregate notwendig ist, um auch bei hohen Verdampfungsgeschwindigkeiten Synthesedampf mit dem höchstmöglichen Trioxangehalt, der dem Gleichgewichtswert in der Gasphase für das Verteilungsgleichgewicht von Trioxan in flüssiger und gasförmiger Phase entspricht, zu erzeugen. Nach früher beschriebenen Verfahren ist dieser Trioxangehalt nur bei Arbeiten mit niederen Verdampfungsgeschwindigkeiten oder nach der in der DE-B-1 543 390 beschriebenen, aufwendigeren Verfahrensweise realisierbar Erfindungsgemäss sind somit Raumzeitausbeu n erreichbar, die wesentlich höher sind als die nach den früher beschriebenen Verfahren erzielten.

Das erfindungsgemässe Verfahren ermöglicht ausserdem eine Trioxansynthese mit einem minimalen Energiebedarf; es weist also eine günstige Energiebilanz auf.

Weiterhin zeichnet sich die Verfahrensweise gemäss der Erfindung dadurch aus, dass durch die kurzen Verweilzeiten bei Anwendung hoher Verdampfungsgeschwindigkeiten, die Bildung von Nebenprodukten wie z.B. Ameisensäure zurückgedrängt wird. In gleicher Weise wirken sich geringe Katalysatorsäure-Konzentrationen oder die Verwendung von Ionenaustauscher aus.

Die folgenden Beispiele sollen das erfindungsgemässe Verfahren näher erläutern.

*Beispiele*

Die verwendete Versuchsapparatur ist in anliegender Figur skizziert. Der Reaktor besteht darin aus dem Kessel (1), dem Verdampfer (2), im speziellen Fall einem Röhrenwärmetauscher und der Pumpe (3). Bei (4) wird wässrige Formaldehydlösung nachdosiert. Der saure Katalysator wird zusammen mit wässriger Formaldehydlösung im Reaktionsgefäss (1) vorgelegt, während bei (5) das Destillat abgezogen wird. (7) bedeutet ein zylindrisches Trennelement zum besseren Stoffaustausch zwischen flüssiger und gasförmiger Phase. Das den Verdampfer verlassende Flüssigkeits-Dampfgemisch wird in den Kessel unterhalb des Flüssigkeitsspiegels über das Tauchrohr (6) eingeleitet. Die Eintauchtiefe des Einleitungsrohres beträgt 70% des Reaktorfüllstandes.

Im Reaktionskessel (1) werden jeweils 1 000g einer Mischung, bestehend aus 90 Teilen einer 63,5%igen wässrigen Formaldehydlösung und 10 Teilen konzentrierter Schwefelsäure vorgelegt. Der Kessel (1) ist auf 95° C temperiert. Das Gemisch wird mittels der Pumpe (3) durch den Verdampfer (2) gepumpt. Der Verdampfer (2) wird je nach dem gewünschten Durchsatz verschieden stark beheizt. Der das System verlassende Synthesedampf wird in einem Quenchsystem total kondensiert. Das Kondensat wird auf Trioxan und Formaldehydgehalt untersucht. Entsprechend dem verdampften Anteil wird 63,5%ige Formaldehydlösung nachdosiert. Die Versuchsdauer beträgt jeweils 6 Stunden. Die Ergebnisse sind zusammen mit den Vergleichsversuchen in Tabelle 1 und Tabelle 2 zusammengestellt.

Man sieht an Tabelle 1 (Beispiel 1–3), dass die Trioxankonzentrationen im Synthesedampf unabhängig vom Verdampfungsgrad im Verdampfer sind.

Tabelle 1

| Beispiel Nr. | Trioxan im Destillat % | Verdampfungsgrad % | Umsatz % | Verweilzeit h |
|---|---|---|---|---|
| 1 | 22,3 | 1,2 | 35,1 | 1,00 |
| 2 | 21,5 | 2,5 | 33,8 | 0,96 |
| 3 | 22,6 | 11,1 | 35,6 | 1,00 |

Aus Tabelle 2 kann man erkennen, dass bei identischen Verweilzeiten die Trioxankonzentration im Synthesedampf sowohl beim erfindungsgemässen Verfahren als auch beim Vergleichsbeispiel (gemäss DE-B-1 543 390) grösser als 20 Gewichtsprozent sind.

Das erfindungsgemässe Verfahren zeichnet sich aber durch wesentlich höhere Raumzeitausbeuten bedingt durch die bedeutend kleineren Reaktionsvolumina als beim Vergleichsverfahren (DE-B-1 543 390) aus.

Tabelle 2

| Beispiel Nr. | Formaldehyd- konzentration Einlauf % | Verweil- zeit h | Trioxan im Destillat % | Reaktorvolumen | Verhältnis der Reaktionsvolumina | Umsatz zu Trioxan % | Verhältnis der Raumzeit- Ausbeuten |
|---|---|---|---|---|---|---|---|
| 4 | 63,5 | 0,3 | 22,4 | Reaktor, Verdampfer, Pumpe, | 1 | 35,3 | 1 |
| 5 * | 63,5 | 0,3 | 20,8 | Reaktor, Pumpe, Verdampfer, Kolonne, lange Rohrleitung | 1,5–2 | 32,8 | 0,68–0,51 |

* gemäss Beispiel 2 der DE-B-1 543 390

**Patentansprüche**

1. Verfahren zur kontinuierlichen Herstellung von Trioxan, gegebenenfalls zusammen mit cyclischen Formalen, aus wässrigen Formaldehyd-Lösungen, die gegebenenfalls mindestens ein Diol und/oder mindestens ein Epoxyd enthalten, in Gegenwart von sauren Katalysatoren in einem Umlaufreaktor mit Verdampfer bei Verweilzeiten von 2 bis 240 Minuten, dadurch gekennzeichnet, dass das den Verdampfer verlassende Dampf-Flüssigkeitsgemisch unter den Flüssigkeitsspiegel der Reaktionsmischung im Reaktor eingeleitet wird und der dampfförmige Produktstrom aus dem Reaktor austritt.

2. Verfahren nach Anspruch 1, dadurch gekennzeichnet, dass das den Verdampfer verlassende Dampf-Flüssigkeitsgemisch 30 bis 70% unterhalb des Flüssigkeitsspiegels eingeleitet wird.

3. Verfahren nach Anspruch 1 und 2, dadurch gekennzeichnet, dass die das System verlassende Dampfmenge, bezogen auf die durch den Verdampfer umlaufende Produktmenge, 6 bis 14% beträgt.

**Claims**

1. A process for the continuous manufacture of trioxan, optionally together with cyclic formals, from aqueous formaldehyde solutions containing optionally at least one diol and/or at least one epoxide, in the presence of acidic catalysts, in a circulation reactor with evaporator, at a residence time in the range of from 2 to 240 minutes, wherein the vapor/liquid mixture leaving the exaporator is fed in below the liquid level of the reaction mixture in the reactor, and the vaporous product current is let off from the reactor.

2. The process as claimed in Claim 1, wherein the vapor/liquid mixture leaving the evaporator is fed in at 30 to 70% below the liquid level.

3. The process as claimed in Claims 1 and 2, wherein the vapor amount leaving the system is from 6 to 14%, relative to the product amount circulating trough the evaporator.

**Revendications**

1. Procédé de préparation continu du trioxanne, éventuellement avec des formals cycliques, à partir de solutions aqueuses de formaldéhyde contenant éventuellement au moins un diol et/ou au moins un époxyde, en présence de catalyseurs acids, dans un réacteur à circulation muni d'un évaporateur, avec des temps de séjour de 2 à 240 minutes, procédé caractérisé en ce que le mélange vapeurliquide sortant de l'évaporateur est introduit au dessous du niveau atteint par le mélange réactionnel liquide dans le réacteur, et le courant de produit à l'état de vapeur sort du réacteur.

2. Procédé selon la revendication 1, caractérisé en ce que le mélange vapeur liquide sortant de l'évaporateur est introduit au-dessous du niveau du liquide à une distance de celui-ci représentant de 30 à 70% de la hauteur de remplissage.

3. Procédé selon l'une des revendications 1 et 2, caractérisé en ce que la quantité de vapeur sortant du système, par rapport à la quantité de produit circulant à travers l'évaporateur, est de 6 à 14%.